(19) Europäisches Patentamt
European Patent Office
Office européen des brevets

(11) Veröffentlichungsnummer: **0 281 012 B1**

(12) **EUROPÄISCHE PATENTSCHRIFT**

(45) Veröffentlichungstag der Patentschrift: **11.12.91**

(51) Int. Cl.[5]: **C07D 263/34**, C07D 413/04, C07D 413/06

(21) Anmeldenummer: **88102772.6**

(22) Anmeldetag: **25.02.88**

# (54) 4-Chloroxazol-Derivate, Verfahren zu ihrer Herstellung und ihre Verwendung.

(30) Priorität: **04.03.87 DE 3706880**

(43) Veröffentlichungstag der Anmeldung:
**07.09.88 Patentblatt 88/36**

(45) Bekanntmachung des Hinweises auf die Patenterteilung:
**11.12.91 Patentblatt 91/50**

(84) Benannte Vertragsstaaten:
**DE FR GB**

(56) Entgegenhaltungen:
**EP-A- 0 010 652**
**DE-A- 3 021 599**
**US-A- 3 819 356**

**CHEMICAL ABSTRACTS, Band 87, Nr. 7, 15. August 1977, Columbus, Ohio, USA; DAVIS, MICHEAL; LAKHAN, RAM; TERNAI, BELA; "A general synthesis of 2,5-diaryl-4-chloro-(or bromo)oxazoles" Seite 457, Spalte 2, Zusammenfassung Nr. 53132x**

(73) Patentinhaber: **HOECHST AKTIENGESELLSCHAFT**
**Postfach 80 03 20**
**W-6230 Frankfurt am Main 80(DE)**

(72) Erfinder: **Wingen, Rainer, Dr. Dipl.-Chem.**
**Rotkäppchenweg 10**
**W-6234 Hattersheim 3(DE)**
Erfinder: **Günther, Dieter, Dr. Dipl.-Chem.**
**Nachtigallenweg 1a**
**W-6233 Kelkheim(DE)**
Erfinder: **Lingnau, Jürgen, Dr. Dipl.-Chem.**
**Karolingerstrasse 10**
**W-6500 Mainz-Laubenheim(DE)**

**EP 0 281 012 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 80, Nr. 13, 1. April 1974, Columbus, Ohio, USA; DRACH, B.S.; DOLGUSHINA, I.YU.; SINITSA, A.D.; "Reaction of condensation products of phenylglyocal and acid amides with thionyl chloride and phosphorus pentachloride" Seite 341, Spalte 1, Zusammenfassung Nr. 70 740s

CHEMICAL ABSTRACTS, Band 76, Nr. 5, 31. Januar 1972, Columbus, Ohio, USA; ZAUGG, HAROLD E.; DE NET, ROBERT W.; "3-Monosubstituted-1-benzoyl-2,2-dichloroaziridines. Methanolysis, thermolysis, and benzoylation" Seite 345, Spalte 2, Zusammenfassung Nr. 24 984u

CHEMICAL ABSTRACTS, Band 84, Nr. 11, 15. März 1976, Columbus, Ohio, USA; IONESCU, MARIA; MACAROVICI, RODICA; BEU, LUCIA; "Preparation and properties of oxazoles. I. Synthesis of halogenated 2,5-diphenyloxazoles" Seite 441, Spalte 1, Zusammenfassung Nr. 74 152g

## Beschreibung

Die Erfindung betrifft neue 4-Chloroxazol-Derivate, Verfahren zu ihrer Herstellung und ihre Verwendung.

4-Chloroxazol-Derivate, deren Herstellung und Verwendung in photoleitfähigen Schichten und als optische Aufheller sind aus EP-B- 0 010 652 bekannt. Die Herstellung erfolgt durch Kondensation eines Acylcyanids mit einem Aldehyd in Gegenwart von Chlorwasserstoff. 4-Chloroxazol-Derivate, die in 2- bzw. 5-Position ungesättigte Gruppen tragen, lassen sich jedoch auf diese bekannte Weise nicht herstellen, da während der Kondensationsreaktion auch Addition von Chlorwasserstoff an die Doppelbindung erfolgt.

Gegenstand der Erfindung sind neue 4-Chloroxazol-Derivate der allgemeinen Formel I

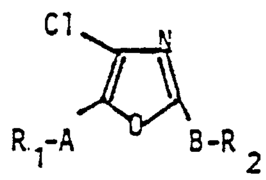

(I),

worin

A eine Einfachbindung oder die -CH=CH- Gruppe,

B eine Einfachbindung, die -CH=CH- oder -CH=CH-CH=CH- Gruppe und dann eine Einfachbindung ist, wenn A die -CH=CH- Gruppe darstellt,

$R_1$ Phenyl, das gegebenenfalls ein- oder mehrfach durch ($C_1$-$C_4$)-Alkoxy- oder Di-($C_1$-$C_4$)-alkylaminoreste substituiert ist, und

$R_2$ Phenyl, Phenyloxazolyl, Pyridyl, Julolidin-9-yl, N-($C_1$-$C_4$-Alkyl)-carbazol-3-yl, Cumarin-6-yl oder Stilben-4-yl, die gegebenenfalls ein- oder mehrfach durch ($C_1$-$C_4$)-Alkyl-, ($C_1$-$C_4$)-Alkoxy-, Halogen-($C_1$-$C_4$)-alkoxy, Hydroxy-, Halogen-, Di-($C_1$-$C_4$)-alkylamino- oder Dibenzylaminoreste substituiert sind,

bedeuten. Bevorzugt sind solche Verbindungen, bei denen in der allgemeinen Formel I

A eine Einfachbindung

B die -CH=CH- Gruppe

$R_1$ Phenyl, das durch ($C_1$-$C_4$)-Alkoxyreste substituiert ist, und

$R_2$ Phenyl oder N-($C_1$-$C_4$-Alkyl)-carbazol-3-yl die durch ($C_1$-$C_4$)-Alkoxy-, Halogen-, Hydroxy- und/oder Di-($C_1$-$C_4$)-alkylaminoreste substituiert sind,

bedeuten. Insbesondere geeignet sind Verbindungen nach der allgemeinen Formel I, bei denen

A eine Einfachbindung,

B die -CH=CH- Gruppe,

$R_1$ Methoxyphenyl und

$R_2$ p-Ethoxyphenyl, p-Dimethylaminophenyl, p-Diethylaminophenyl, o-Chlor-p-dimethylaminophenyl, o-Hydroxy-p-diethylaminophenyl oder N-Ethylcarbazol-3-yl bedeuten.

Die erfindungsgemäßen 4-Chloroxazol-Derivate nach der allgemeinen Formel I können im Prinzip dadurch hergestellt werden,daß man die ungesättigten Strukturelemente A bzw. B zusammen mit den jeweiligen Substituenten $R_1$ bzw. $R_2$ mit einer entsprechenden 4-Chloroxazolverbindung durch Kondensation in Gegenwart einer Base miteinander verbindet und das 4-Chloroxazol-Derivat aus dem Reaktionsgemisch isoliert und trocknet.

Gegenstand der Erfindung ist demgemäß ein Verfahren zur Herstellung einer Verbindung nach der allgemeinen Formel I, in der

A eine Einfachbindung und

B die -CH=CH- Gruppe

bedeuten, bei dem man eine Verbindung nach Formel II

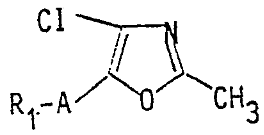

(II)

mit der für $R_1$ angegebenen Bedeutung mit einer Verbindung der Formel III

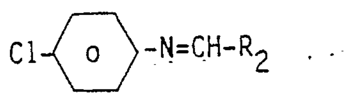

(III)

mit der für $R_2$ angegebenen Bedeutung, in einem inerten organischen Lösungsmittel in Gegenwart einer Base bei einer Temperatur im Bereich von 20 bis 80 °C kondensiert, abtrennt, trocknet und die Verbindung gegebenenfalls durch Umfällen reinigt (Verfahren A). Vorzugsweise kondensiert man bei einer Temperatur von 40 bis 60 °C, verwendet als Lösungsmittel Dimethylformamid und als Base Kaliumhydroxid.

Gegenstand der Erfindung ist auch ein Verfahren zur Herstellung einer Verbindung nach der allgemeinen Formel I, in der

A die -CH=CH- Gruppe und

B eine Einfachbindung

bedeuten,

bei dem man eine Verbindung nach der Formel IV

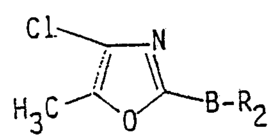

(IV)

mit der für $R_2$ angegebenen Bedeutung mit einer Verbindung der Formel V

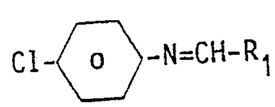

(V)

mit der für $R_1$ angegebenen Bedeutung in einem inerten organischen Lösungsmittel in Gegenwart einer Base bei einer Temperatur im Bereich von 0 bis 40 °C kondensiert, abtrennt, trocknet und die Verbindung gegebenenfalls durch Umfällen reinigt. Vorzugsweise kondensiert man bei einer Temperatur von 20 - 30 °C (Verfahren B).

Gegenstand der Erfindung ist schließlich auch ein Verfahren zur Herstellung einer Verbindung nach der allgemeinen Formel I, in der

A eine Einfachbindung und

B die -CH = CH- oder die -CH = CH-CH = CH- Gruppe

bedeuten,

bei dem man ein Phosphoniumsalz nach der Formel VI

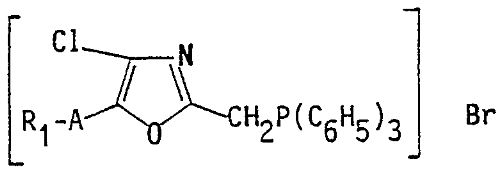

(VI)

mit der für $R_1$ angegebenen Bedeutung, mit einem Aldehyd nach der Formel VII

X - $R_2$ (VII)

mit der für $R_2$ angegebenen Bedeutung und mit X als -CHO oder -CH = CH-CHO in einem inerten organischen Lösungsmittel in Gegenwart einer Base bei Raumtemperatur zu einer Verbindung kondensiert, vom Reaktionsgemisch abtrennt, trocknet und gegebenenfalls durch Umfällen reinigt (Verfahren C). Als Lösungsmittel wird vorzugsweise Dimethylsulfoxid und als Base Dimethylsulfinylnatrium verwendet.

Die Verknüpfung erfolgt durch Kondensation von 2-Methyl-4-chloroxazolen der Formel II (Verfahren A) oder von 5-Methyl-4-chloroxazolen der Formel IV (Verfahren B) mit geeigneten Derivaten (III bzw. V) von Aldehyden in für Methyl-Heterocyclen allgemein literaturbekannter Weise (I.J.Fletcher, A.E.Sigrist in "Adv. Heterocyclic Chem.", A.R. Katritzky, A.J.Bowlton, eds., Academic Press, New York, 1978, Vol. 23, p.171).

Vorteilhaft wird auch die Kondensation von Aldehyden VII mit Triphenylphosphoniumverbindungen des 4-Chlor-oxazols VI durchgeführt unter Bedingungen, wie sie im Rahmen von Wittig-Reaktionen (Neuere Methoden der Organischen Chemie, W.Foerst Hrsg.,Verlag Chemie Weinheim, 1967, Bd. V.,S. 1) üblich sind (Verfahren C).

<u>Schema 1</u>

Verfahren A

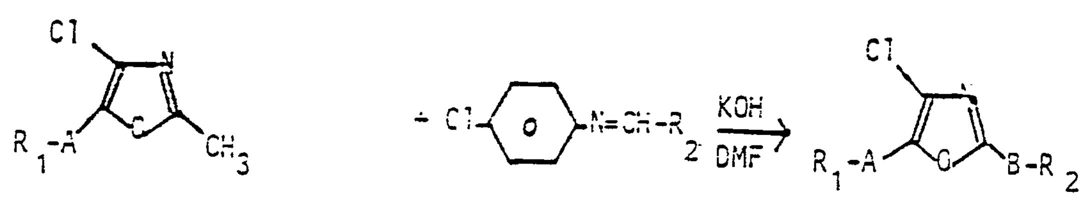


II III I

Schema 2

Verfahren B

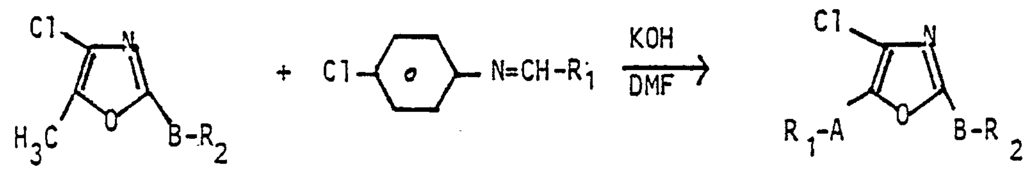


IV V I

Schema 3

Verfahren C

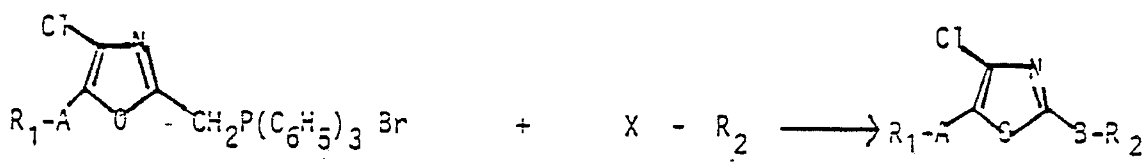

VI VII I

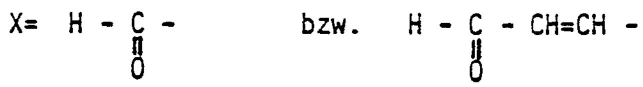

Die als Ausgangsmaterialien benötigten 4-Chloroxazole II bzw. IV lassen sich nach literaturbekannten Verfahren, wie sie in der EP-B- 0 010 652 aufgeführt werden, leicht erhalten, beispielsweise durch Chlorwasserstoffkatalysierte Kondensation von substituierten Benzoylcyaniden mit Acetaldehyd (für II) oder durch analoge Reaktion von Acetylcyanid (Brenztraubensäurenitril) mit Aldehyden (für IV).

Die Verbindungen VI sind in literaturbekannter Weise (Houben-Weyl, Methoden der Organischen Chemie, Bd. V, 1b, Seite 383, E.Müller Hrsg., G.Thieme Verlag, 1972) aus den entsprechenden Brommethyl-oxazolen erhältlich, die ihrerseits, z.B. durch Bromieren der Verbindungen II oder IV leicht zugänglich sind.

Von besonderem Interesse sind die Verbindungen der allgemeinen Formel I als photoleitfähige Substanzen, beispielsweise in elektrophotographischen Aufzeichnungsmaterialien. Solche bestehen aus einer leitfähigen Unterlage und einem Schichtaufbau, der im Dunkeln isolierend ist, aber unter Belichtung leitend wird. Der Aufbau kann aus einer oder mehreren Schichten bestehen. Im Fall einer einzigen Schicht ist mindestens eine photoleitfähige Substanz in mindestens einem Bindemittel dispergiert und unmittelbar auf eine leitende Unterlage aufgedampft. Ein mehrschichtiger Aufbau besteht aus mindestens einer ladungsträgererzeugenden und mindestens einer ladungstransportierenden Schicht.

Die Erzeugung von Ladungsbildern auf elektrophotographischen Schichten erfolgt nach der Aufladung durch einen Belichtungsschritt. Durch Belichtung wird zunächst elektrische Ladung erzeugt, die in einem weiteren Schritt durch die Schicht hindurch bis an die Schichtoberfläche transportiert wird und dort vorhandene Ladung neutralisiert. Dabei unterscheidet man Photorezeptoren, bei denen Ladungserzeugung und Ladungstransport durch die gleiche chemische Substanz bewirkt werden, von solchen, bei denen die

Ladungserzeugung durch Zugabe einer zweiten Substanz, des Sensibilisators, erreicht wird. Dadurch wird die Möglichkeit gegeben, Elektrophotographie auch mit Licht einer Wellenlänge zu betreiben, das von der eigentlich photoleitfähigen Substanz nicht absorbiert wird.

Zur homogenen Sensibilisierung werden die in den Farbstofftabellen von Schultz (7. Auflage, 1. Band, 1931) aufgeführten organischen Farbstoffe eingesetzt, zum Beispiel Triarylmethanfarbstoffe, wie Brillantgrün (Nr. 760, S. 314), Victoriablau B (Nr. 822, S. 347), Methylviolett (Nr. 783, S. 327), Kristallviolett (Nr. 785, S. 329), Säureviolett 6B (Nr. 381, S. 351); Xanthenfarbstoffe, und zwar Rhodamine, wie Rhodamin B (Nr. 864, S. 365), Rhodamin 6G (Nr. 866, S. 366), Rhodamin G extra (Nr. 865, S. 366), Sulforhodamin B (Nr. 863, S. 364) und Echtsäureeosin G (Nr. 870, S. 368) sowie Phthaleine, wie Eosin S (Nr. 883, S. 375), Eosin A (Nr. 881, S. 374), Erythrosin (Nr. 866, S. 376), Phloxin (Nr. 890, S. 378), Rose bengale (Nr. 889, S. 378) und Fluorescein (Nr. 880, S. 373); Thiazinfarbstoffe, wie Methylenblau (Nr. 1038, S. 449); Acridinfarbstoffe, wie Acridingelb (Nr. 901, S. 383), Acridinorange (Nr. 908, S. 387) und Trypaflavin (Nr. 906, S. 386); Chinolinfarbstoffe, wie Pinacyanol (Nr. 924, S. 396) und Kryptocyanin (Nr. 927, S. 397); Chinonfarbstoffe und Ketonfarbstoffe, wie Alizarin (Nr. 1141, S. 449), Alizarinrot S (Nr. 1145, S. 502) und Chinalizarin (Nr. 1148, S. 504); Cyaninfarbstoffe (Polymethinfarbstoffe), wie Astrazongelb 3G (Colour Index Nr. (C.I.) 48055) und 5 G (C.I. 48 065), Basic Yellow 52115 (C.I. 48 060), Astrazongelb GRL, Astrazonorange G (C.I. 48 040) und R (C.I. 48 035), Astrazonorange 3R (noch nicht klassifiziert).

Pyryliumsalze, Thiapyryliumsalze, Benzopyryliumsalze können ebenfalls verwendet werden.

Sensibilisierungsfarbstoffmischungen können ebenfalls vorliegen.

Als dispers verteilte Farbstoffe oder Pigmente können auch metallhaltige oder metallfreie Phthalocyaninpigmente, zum Beispiel Kupferphthalocyanin, Perinon-, Thioindigo-, höher annellierte Chinon-, Chinacridon-, Perylen-, Anthrachinon-, Dioxazin-, Azo-, Bisazo-, Trisazo-, Cyanin-Pigmente oder Benzo(thio)-xanthen-Derivate sowie deren Mischungen eingesetzt werden. Davon sind besonders bevorzugt Phthalocyaninpigmente, wie die verschiedenen Cu-Phthalocyaninmodifikationen ($\alpha$, $\beta$, $\epsilon$), Bis- und Trisazopigmente, Perylen-3,4,9,10-tetracarbonsäureanhydrid und seine Imidderivate, oder (Iso)Violanthrone. Sie können bis zu 30 %, bezogen auf die Gesamtmasse der photoleitfähigen Schicht, vorhanden sein, bevorzugt werden Mengen im Bereich von 0,1 bis 10 % Pigment verwendet.

Das hochisolierende Bindemittel für die ladungsträgererzeugende Schicht und für die Ladungstransportschicht kann gleich oder unterschiedlich sein. Als solche sind hinsichtlich der Flexibilität, der Filmeigenschaften und der Haftfestigkeit Natur- und Kunstharze geeignet, die sich durch gebräuchliche Lösungsmittel oder Lösungsmittelgemische bei der Herstellung der Schichten anlösen bzw. anquellen lassen. Hierzu gehören Polyesterharze, die Mischpolyester aus Iso- und Terephthalsäure mit Glykolen darstellen. Auch Silikonharze haben sich als geeignet erwiesen. Polycarbonatharze sind gut einsetzbar. Besonders bevorzugt für die Herstellung von Druckformen und gedruckten Schaltungen sind Bindemittel, die in wäßrigen oder alkoholischen Lösungsmittelsystemen, gegebenenfalls unter Säure- oder Alkalizusatz, löslich sind. Aus physiologischen und Sicherheitsgründen scheiden aromatische oder aliphatische, leicht brennbare Lösungsmittel aus. Geeignete Harzbindemittel sind hiernach hochmolekulare Substanzen, die alkalilöslich machende Gruppen tragen. Solche sind beispielsweise Säureanhydrid-, Carboxyl-, Carbonsäureamid-, Phenol-, Sulfonsäure-, Sulfonamid-oder Sulfonimid-Gruppen. Bevorzugt werden Harzbindemittel mit hohen Säurezahlen eingesetzt. Mischpolymerisate mit Anhydridgruppen können mit gutem Erfolg verwendet werden, da durch das Fehlen freier Säuregruppen die Dunkelleitfähigkeit gering ist, trotz guter Alkalilöslichkeit. Besonders bewährt haben sich Copolymerisate aus Styrol und Maleinsäureanhydrid, Sulfonylurethane gemäß DE-OS 32 10 577, und Copolymerisate der Acryl- bzw. Methacrylsäure.

Als übliche Zusätze enthalten die Schichten Substanzen, die der Beschichtungslösung zugesetzt werden und dadurch die Oberflächenstruktur und die Flexibilität verbessern. Dies können zum Beispiel Weichmacher, wie Triphenylphosphat, oder Verlaufmittel, wie Silikonöle, sein.

Als elektrisch leitende Schichtträger sind Materialien mit genügend elektrisch leitenden Eigenschaften geeignet,wie sie auch bisher bereits zu diesem Zweck verwendet wurden. Der Schichtträger kann in Form eines flexiblen Bandes oder einer Platte vorliegen. In bevorzugter Ausführungsform ist der Schichtträger für die Herstellung von Druckformen und gedruckten Schaltungen geeignet und besteht zum Beispiel aus einer Aluminium-, Zink-, Magnesium-, Kupfer-, Eisen-, Nickel- oder einer Mehrmetallplatte. Es kommen auch metallisierte, zum Beispiel metallbedampfte Kunststoffolien, wie aluminiumbedampfte Polyesterfolien, oder auch kupferkaschierte Polyimidfolien und -platten in Frage.

Besonders bewährt haben sich oberflächenveredelte Schichtträger aus Aluminium. Die Oberflächenveredlung besteht in einer mechanischen oder elektrochemischen Aufrauhung und gegebenenfalls in einer anschließenden Anodisierung und Behandlung mit Polyvinylphosphonsäure gemäß DE-OS 16 21 478, entsprechend US-PS 4,153,461.

Ganz allgemein kann eine isolierende Zwischenschicht auf dem Schichtträger vorhanden sein, wie eine

thermisch, anodisch bzw. chemisch erzeugte Metalloxidschicht, zum Beispiel aus Aluminiumoxid. Diese Sperrschicht hat die Aufgabe, die Ladungsträgerinjektion vom elektrisch leitenden Schichtträger im Dunkeln in die Ladungsträger erzeugende Schicht herabzusetzen bzw. zu verhindern. Weiterhin wird durch die Sperrschicht die Haftung der folgenden Schichten auf dem Schichtträger günstig beeinflußt. Für organische Sperrschichten können verschiedene Natur-oder Kunstharzbindemittel verwendet werden, die gut auf einer Metall-bzw. Aluminiumoberfläche haften und beim nachfolgenden Anbringen der weiteren Schichten keine An- oder Ablösung erfahren. Die Dicke der organischen Sperrschicht liegt im Bereich von 1 µm, die einer Metalloxidschicht in der Größenordnung von 1o bis $1o^4$ nm.

Zur Herstellung von beispielsweise gedruckten Schaltungen, wie sie in der Elektronik üblich sind, kann die photoleitfähige Schicht auch zunächst auf einen Zwischenträger aufgebracht werden, von dem aus sie als sogenannter Trockenresist auf den Schichtträger anschließend oder später übertragen wird. Dies kann zum Beispiel durch Laminieren erfolgen. Als Zwischenträger haben sich Kunststoffolien, wie solche aus Polyester, insbesondere aus Polyethylenterephthalatfolie, besonders bewährt.

Die Beschichtungen bringt man in üblicher Weise auf, zum Beispiel durch Rakel- oder Sprühantrag. Vorzugsweise wird der Antrag mit einem Fließer vorgenommen. Die Trocknung der Schichten erfolgt beispielsweise in Trockenkanälen, wobei die verschiedenen Trocknungsstufen durch die Temperatur der einzelnen Bereiche, durch die Laufgeschwindigkeit des Materials und durch den herrschenden Luftdurchsatz festgelegt werden.

Die Synthese der Verbindungen nach der allgemeinen Formel I nach den jeweiligen Verfahren A, B oder C wird im Nachfolgenden durch allgemeine Arbeitsvorschriften näher erläutert; die dargestellten Verbindungen I sind in Tabelle 1 zusammengefaßt aufgeführt.

Verfahren A - allgemeine Vorschrift

Eine Lösung von 4o mmol 2-Methyl-4-chlor-oxazol II und 50 mmol N-(4-Chlor-phenyl)-$R_2$ -azomethin III in 1oo ml Dimethylformamid wird mit 160 mmol Kaliumhydroxid-Pulver versetzt. Binnen 30 min wird auf 60° C erhitzt und 2h bei dieser Temperatur gehalten. Nach Abkühlung auf 20° C wird mit 400 ml Methanol versetzt, auf -10° C gekühlt, der Feststoff abgetrennt und aus Methylglykol umkristallisiert.

Elementaranalyse für

1-[4-Chlor-5-(4-methoxy)phenyl-oxazol-2-yl]-2-(4-dimethylamino)-phenyl-ethen, wobei in der allgemeinen Formel I

- $R_1$ 4-Methoxyphenyl
- A Bindung
- B -CH=CH-
- $R_2$ 4-Dimethylaminophenyl bedeuten:

| | | | | | |
|---|---|---|---|---|---|
| $C_{20}H_{19}ClN_2O_2$ | ber. | C 67.7 | H 5.4 | Cl 10.0 | N 7.9 |
| | gef. | 67.8 | 5.3 | 10.3 | 7.9 |

Verfahren B - allgemeine Vorschrift

Eine Lösung von 17 mmol 5-Methyl-4-chloroxazol IV, 21 mmol N-(4-chlor-phenyl)-$R_1$-azomethin V und 68 mmol Kaliumhydroxid-Pulver in 40 ml Dimethylformamid wird 2h bei 20° C gerührt. Nach Zugabe von 160 ml Methanol wird auf -10° C gekühlt, der Feststoff abgetrennt und aus Methylglykol umkristallisiert.

Elementaranalyse für

1-[4-Chlor-2-(4-dimethylamino)phenyl-oxazol-5-yl]-2-(4-dimethyl-amino)-phenyl-ethen, wobei in der allgemeinen Formel I

- $R_1$ 4-Dimethylaminophenyl
- A -CH=CH-
- B Bindung

$R_2$ 4-Dimethylaminophenyl bedeuten:

| | | | | | |
|---|---|---|---|---|---|
| $C_{21}H_{22}ClN_3O$ | ber. | C 68.6 | H 6.0 | Cl 9.6 | N 11.4 |
| | gef. | 68.4 | 6.1 | 9.7 | 11.4 |

Verfahren C - allgemeine Vorschrift

60 mmol Natriumhydrid (80 %-ige Suspension) und 100 ml Dimethylsulfoxid werden, zuletzt durch Erhitzen auf 75° C, zur Reaktion gebracht. Die abgekühlte Lösung wird mit 60 mmol fein gepulvertem Triphenylphosphoniumsalz VI versetzt und nach erfolgter Auflösung noch 50 min bei 25° C gerührt. 60 mmol Aldehyd VII werden zugetropft und die Mischung noch 2 h bei 25° C sowie 3 h bei 75° C gerührt. Der nach Hydrolyse mit 500 ml Eiswasser erhaltene Feststoff wird aus Methylglykol umkristallisiert.

Elementaranalyse für

1-[4-Chlor-5-phenyl-oxazol-2-yl]-2-[4-chlor-5-(4-methoxy)-phenyl-oxazol-2-yl]-ethen, wobei in der allgemeinen Formel I

$R_1$ Phenyl
A Bindung
B -CH=CH-
$R_2$ 4-Chlor-5-(4-methoxy)phenyl-oxazol-2-yl bedeuten:

| | | | | | |
|---|---|---|---|---|---|
| $C_{21}H_{14}Cl_2N_2O_3$ | ber. | C 61.0 | H 3.4 | Cl 17.2 | N 6.8 |
| | gef. | 60.8 | 3.5 | 17.6 | 6.6 |

Vergleichsbeispiel 1

Nach der in der EP-B-0 010 652 gegebenen Vorschrift wird eine Lösung von 100 mmol 4-Methoxybenzoylcyanid und 100 mmol Zimtaldehyd in 5o ml Tetrahydrofuran bei 0° C mit Chlorwasserstoff gesättigt. Nach 10-stündigem Stehen bei 0° C wird auf 200g Eis gegeben, mit Dichlormethan extrahiert und der Extrakt gewaschen und getrocknet. Durch Säulenchromatographie an Kieselgel bei Elution durch Dichlormethan läßt sich eine Chloroxazol-haltige Fraktion als Hauptbestandteil abtrennen.
Nach gaschromatographischer Analyse ist darin das ungesättigte Kondensationsprodukt, entsprechend der auf anderem Weg hergestellten Verbindung Ig, nur zu 19,4 % enthalten.
Hauptbestandteil mit zusammen 68,1 % sind Verbindungen, die nach $^1$H-NMR-Analyse eine 1,2-substituierte Chlorethyl-Einheit enthalten

Trennung dieser Mischung mittels Chromatographie, Destillation oder Kristallisation erwies sich als nicht praktikabel.

Anwendungsbeispiele:

Die Ausprüfung der elektrophotographischen Eigenschaften der erfindungsgemäßen Verbindungen nach der allgemeinen Formel I erfolgte in unterschiedlichen Schichtrezepturen.

Beschichtungsrezeptur 1:

15.0 g Hostapermorange GR (Pigment Orange 43, C.I. 71.105) wurden in eine Lösung von 10 g Polybutylmethacrylat (® Plexigum P676, Röhm GmbH) in 2oo g Tetrahydrofuran eingetragen und durch Mahlen in einer Kugelmühle während 2 Stunden dispergiert. Nach Zusatz von 32 g Polymethylmethacrylat (® Plexigum M345) in 34o g Tetrahydrofuran wurde die Schicht mit einem Schichtgewicht von 6 g/m$^2$ auf

aluminiumbedampfte Polethylenterephthalatfolie aufgetragen und getrocknet. Die getrocknete Schicht wurde dann mit einer 5 %-igen Lösung der erfindungsgemäßen Verbindungen in THF behandelt und erneut getrocknet.

Beschichtungsrezeptur 2:

5 g der erfindungsgemäßen Verbindungen, 5 g eines Copolymeren aus Maleinsäurehalbester und Styrol, Zersetzungspunkt 200 bis 240 °C, und 0,05 g Rhodamin B werden in 90 g Tetrahydrofuran gelöst und so auf einen aufgerauhten und anodisierten Aluminium-Offsetdruckplattenträger aufgebracht, daß ein Trockenschichtgewicht von 6 $g/m^2$ resultiert.

Beschichtungsrezeptur 3:

Auf eine aluminiumbedampfte Polyethylenterephthalatfolie wurde zunächst eine ladungsträgererzeugende Schicht aus N,N'-Dimethylperylen-3,4,9,10-tetracarbonsäurediimid in einem Schichtgewicht von 200 $mg/m^2$ durch Sublimation aufgetragen. Auf diese Schicht wurde eine Lösung aus 50 Gewichtsteilen der erfindungsgemäßen Substanzen und 50 Gewichtsteilen eines Polyesters (® Dynapol L2o6, Dynamit Nobel) so aufgetragen, daß ein Schichtgewicht dieser Ladungstransportschicht von 10.5 $g/m^2$ resultierte.

Die Resultate der elektrophotographischen Untersuchungen der gemäß den Beispielen hergestellten Schichten sind in der folgenden Tabelle 2 zusammengefaßt. Dabei bedeuten $E_{1/2}$, $E_{1/4}$ und $E_{1/8}$ die Belichtungsenergien in $\mu J/cm^2$, die bei einer Lichtintensität von 3 $\mu W/cm^2$ aufgebracht werden müssen, um eine Entladung von der ursprünglichen Aufladung $U_o$ auf 0.5 $U_o$, 0.25 $U_o$ bzw. 0.125 $U_o$ zu bewirken. Die Messungen der Proben gemäß Schichtrezeptur 1 erfolgten bei 485 nm, die der Proben gemäß Schichtrezeptur 2+3 bei Weißlicht (Halogen-Wolfram-Lampe, Kantenfilter bei 650nm). $U_e$ bedeutet die nach 10 sec Belichtung auf der Schicht verbleibende Restladung.

Vergleichsbeispiele:

Zum Vergleich werden die Beschichtungsrezepturen auch unter Verwendung der Verbindungen IX (DE-PS 1 058 836) bzw. VIII (EP-B 0 010 652 ) untersucht. Bei Beschichtungsrezeptur 1 wurde zum Vergleich auch auf die Nachbehandlung mit einer photoleiterhaltigen Lösung verzichtet.

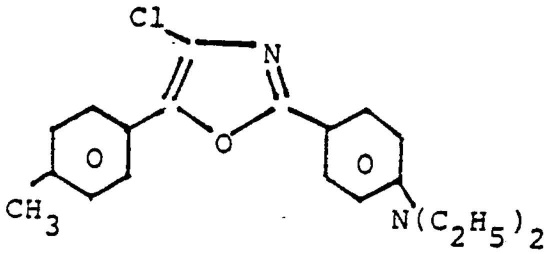

VIII

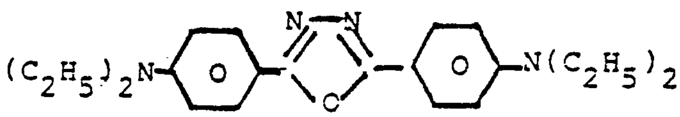

IX

TABELLE 2

| Verb. | Rez. | $U_0$ | $U_e$ | $E_1/_2$ | $E_1/_4$ | $E_1/_8$ |
|---|---|---|---|---|---|---|
| Iac | 1 | -406 | -63 | 16.4 | 25 | |
| Iad | 1 | -462 | -47 | 11 | 14 | 32 |
| Ie | 1 | -430 | -67 | 21.6 | 70 | |
| If | 1 | - 39 | -15 | 70.6 | | |
| Ih | 1 | -770 | -39 | 6.1 | 8 | 13 |
| Ii | 1 | -857 | -47 | 5.7 | 7 | 10 |
| In | 1 | -734 | -31 | 5.9 | 8 | 11 |
| Ir | 1 | -857 | -23 | 3.7 | 4.7 | 6.1 |
| Is | 1 | -647 | -23 | 3.6 | 5.2 | 8.2 |
| Iw | 1 | -632 | -47 | 9 | 23 | 67 |
| Iy | 1 | -193 | -51 | 59.5 | | |
| Iz | 1 | -308 | -59 | 27.5 | 96 | |
| ohne | 1 | -746 | -319 | 75 | | |
| VIII | 1 | -525 | -75 | 20.4 | 62 | |
| IX | 1 | -620 | - 7 | 5.1 | 7 | 10 |
| Iac | 2 | -268 | -31 | 20.7 | 51 | |
| Iad | 2 | -165 | -15 | 19.7 | 50 | 99 |
| Iaf | 2 | -529 | -177 | 70.4 | | |
| Ie | 2 | -481 | -240 | 146 | | |
| If | 2 | -493 | - 51 | 16.9 | 47 | 126 |
| Ig | 2 | -623 | -470 | | | |
| Ih | 2 | -596 | - 55 | 12 | 25 | 47 |
| Ij | 2 | -722 | -481 | | | |
| Il | 2 | -169 | - 3 | 19.1 | 41 | 65 |
| Io | 2 | -292 | - 3 | 10.7 | 27 | |
| Ip | 2 | -730 | - 94 | 11.7 | 28 | |
| Iw | 2 | -647 | - 7 | 7.9 | 18 | 36 |
| Iz | 2 | -406 | -165 | 101 | | |
| VIII | 2 | -398 | - 11 | 10.6 | 30 | 62 |
| IX | 2 | -426 | - 7 | 8 | 20 | 39 |
| Ik | 3 | -647 | -181 | 4.7 | | |
| Im | 3 | -556 | 0 | 1.7 | 3.2 | 5 |
| It | 3 | -339 | 0 | 2.3 | 4.8 | 7.5 |
| IX | 3 | -205 | - 7 | 4.9 | 10.1 | 18.4 |

TABELLE 1

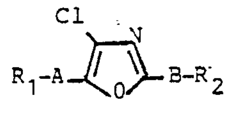

I

| Nr. | A | $R_1$ | B | $R_2$ | Smp [°C] | Verf. |
|---|---|---|---|---|---|---|
| I a | Bindung | 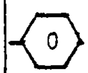 | -CH=CH- | 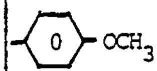 | 128-129 | A |
| b | " | " | " | 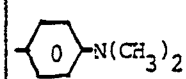 | 124-125 | A |
| c | " | " | " | 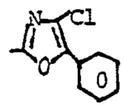 | 188-189 | C |
| d | " | " | " | 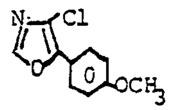 | 175-176 | C |
| e | " | " | -CH=CH-CH=CH- | 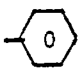 | 99-100 | C |
| f | " | " | " | 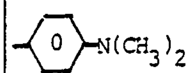 | 142-143 | C |
| g | " | 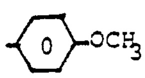 | -CH=CH- | 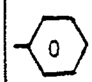 | 106-107 | C |
| h | " | " | " | 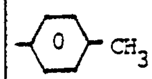 | 124-125 | C |
| i | " | " | " | 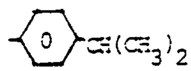 | 76-77 | C |
| j | " | " | " | 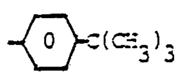 | 91-92 | C |
| k | " | " | " | 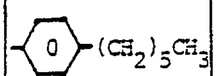 | 67-68 | C |
| l | " | " | " | 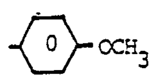 | 159-160 | A |
| m | " | " | " | 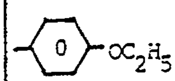 | 130-131 | C |
| n | " | " | " | 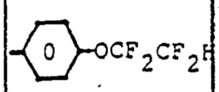 | 104-105 | C |

| Nr. | A | R 1 | B | R 2 | Smp [°C] | Verf. |
|---|---|---|---|---|---|---|
| o | " | " | " | 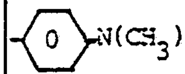 | 171-172 | A |
| p | " | " | " | 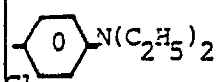 | 107-108 | C |
| q | " | " | " | 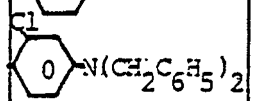 | 143-144 | C |
| r | " | " | " | 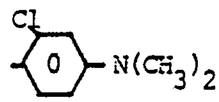 | 166-169 | C |
| s | " | " | " | 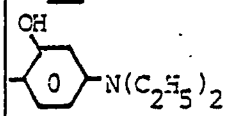 | 210-211 | C |
| t | " | " | " | 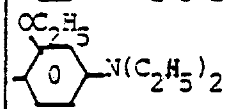 | 92-95 | C |
| u | " | " | " | 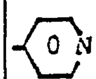 | 134-135 | C |
| v | " | " | " | 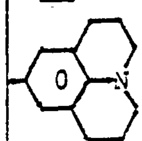 | 137-139 | C |
| w | " | " | " | 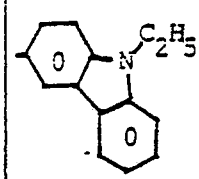 | 175-176 | C |
| x | " | " | " | 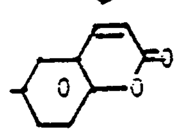 | 211-212 | C |
| y | " | " | " | 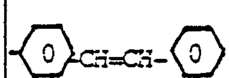 | 193-194 | C |
| z | " | " | -CH=CH-CH=CH- | 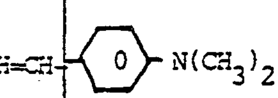 | 158-160 | C |

| Nr. | A | $R_1$ | B | $R_2$ | Smp [°C] | Verf. |
|---|---|---|---|---|---|---|
| aa | " | 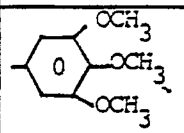 | -CH=CH- | 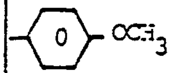 | 138-139 | C |
| ab | " | " | " | 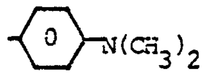 | 205-207 | C |
| ac | " | 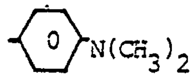 | " | 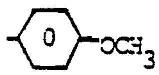 | 138-139 | A |
| ad | " | " | " | 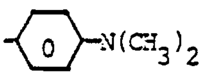 | 154-155 | A |
| ae | " | " | " | 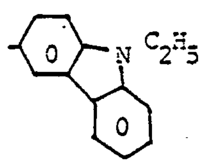 | 178-180 | B |
| af | -CH=CH- | 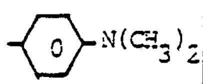 | Bindung | 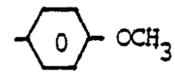 | 164-165 | B |
| ag | " | " | " | 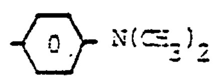 | 195-196 | B |

## Patentansprüche

1. 4-Chloroxazol-Derivate der allgemeinen Formel I

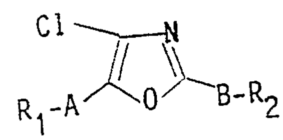

(I),

worin

A eine Einfachbindung oder die -CH=CH- Gruppe,

B eine Einfachbindung, die -CH=CH- oder -CH=CH-CH=CH- Gruppe und dann eine Einfachbindung ist, wenn A die -CH=CH- Gruppe darstellt,

$R_1$ Phenyl, das gegebenenfalls ein- oder mehrfach durch ($C_1$-$C_4$)-Alkoxy- oder Di-($C_1$-$C_4$)-alkylaminoreste substituiert ist, und

$R_2$ Phenyl, Phenyloxazolyl, Pyridyl, Julolidin-9-yl, N-($C_1$-$C_4$-Alkyl)-carbazol-3-yl, Cumarin-6-yl

oder Stilben-4-yl, die gegebenenfalls ein-oder mehrfach durch $(C_1-C_4)$-Alkyl-, $(C_1-C_4)$-Alkoxy-, Halogen-$(C_1-C_4)$-alkoxy, Hydroxy-, Halogen-, Di-$(C_1-C_4)$-alkylamino- oder Dibenzylaminoreste substituiert sind,

bedeuten.

**2.** Verbindungen nach Anspruch 1,

worin
- A eine Einfachbindung,
- B die -CH=CH- Gruppe,
- $R_1$ Phenyl, das durch $(C_1-C_4)$-Alkoxyreste substituiert ist, und
- $R_2$ Phenyl oder N-$(C_1-C_4$-Alkyl)-carbazol-3-yl, die durch $(C_1-C_4)$-Alkoxy-, Halogen-, Hydroxy- und/ oder Di-$(C_1-C_4)$-alkylaminoreste substituiert sind,

bedeuten.

**3.** Verbindungen nach Anspruch 1 oder 2,

worin
- A eine Einfachbindung,
- B die -CH=CH- Gruppe,
- $R_1$ Methoxyphenyl und
- $R_2$ p-Ethoxyphenyl, p-Dimethylaminophenyl, p-Diethylaminophenyl, o-Chlor-p-dimethylaminophenyl, o-Hydroxy-p-diethylaminophenyl oder N-Ethylcarbazol-3-yl

bedeuten.

**4.** Verfahren zur Herstellung einer Verbindung nach der allgemeinen Formel I gemäß Anspruch 1, in der

- A eine Einfachbindung und
- B die -CH=CH- Gruppe

bedeuten, bei dem man eine Verbindung nach Formel II

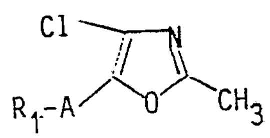

(II)

mit der für $R_1$ angegebenen Bedeutung mit einer Verbindung der Formel III

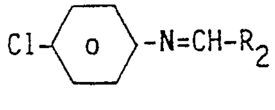

(III)

mit der für $R_2$ angegebenen Bedeutung, in einem inerten organischen Lösungsmittel in Gegenwart einer Base bei einer Temperatur im Bereich von 20 bis 80 °C kondensiert, abtrennt, trocknet und die Verbindung gegebenenfalls durch Umfällen reinigt.

5. Verfahren zur Herstellung einer Verbindung nach der allgemeinen Formel I gemäß Anspruch 1, in der

A die -CH=CH- Gruppe und
B eine Einfachbindung

bedeuten,
bei dem man eine Verbindung nach der Formel IV

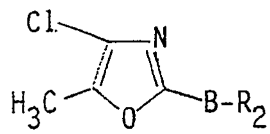

(IV)

mit der für $R_2$ angegebenen Bedeutung mit einer Verbindung der Formel V

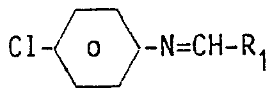

(V)

mit der für $R_1$ angegebenen Bedeutung in einem inerten organischen Lösungsmittel in Gegenwart einer Base bei einer Temperatur im Bereich von 0 bis 40 °C kondensiert, abtrennt, trocknet und die Verbindung gegebenenfalls durch Umfällen reinigt.

6. Verfahren zur Herstellung einer Verbindung nach der allgemeinen Formel I gemäß Anspruch 1, in der

A eine Einfachbindung und
B die -CH=CH- oder die -CH=CH-CH=CH- Gruppe

bedeuten,
bei dem man eine Verbindung der Formel VI

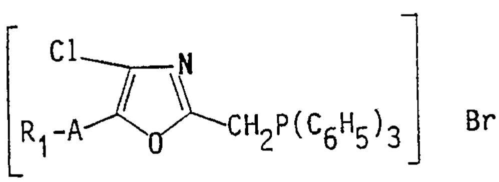

(VI)

mit der für $R_1$ angegebenen Bedeutung, mit einer Verbindung der Formel VII

$X - R_2$ (VII)

mit der für $R_2$ angegebenen Bedeutung und mit

X als -CHO oder -CH = CH-CHO

in einem inerten organischen Lösungsmittel in Gegenwart einer Base bei Raumtemperatur kondensiert, sie vom Reaktionsgemisch abtrennt, trocknet und die Verbindung gegebenenfalls durch Umfällen reinigt.

**7.** Verwendung der Verbindungen der allgemeinen Formel I als photoleitfähige Substanzen.

## Claims

**1.** 4-Chlorooxazole derivatives of the general formula I

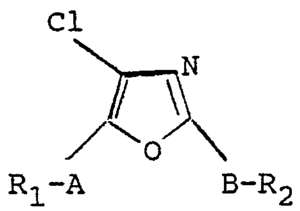

(I)

wherein

A denotes a single bond or the -CH = CH- group,

B denotes a single bond, the -CH = CH- or -CH = CH-CH = CH- group and is a single bond, in the case of A representing the -CH = CH- group,

$R_1$ denotes phenyl, which is optionally substituted in one or more positions by ($C_1$-$C_4$)-alkoxy or di-($C_1$-$C_4$)-alkylamino radicals, and

$R_2$ denotes phenyl, phenyloxazolyl, pyridyl julolidin-9-yl, N-($C_1$-$C_4$-alkyl)-carbazol-3-yl, coumarin-6-yl or stilben-4-yl, which are optionally substituted in one or more positions by ($C_1$-$C_4$)-alkyl, ($C_1$-$C_4$)-alkoxy, halogen-($C_1$-$C_4$)-alkoxy, hydroxy, halogen, di-($C_1$-$C_4$)-alkylamino or dibenzylamino radicals.

**2.** Compounds as claimed in claim 1, wherein

A denotes a single bond,

B denotes the -CH = CH- group,

$R_1$ denotes phenyl, which is substituted by ($C_1$-$C_4$)-alkoxy radicals, and

$R_2$ denotes phenyl or N-($C_1$-$C_4$-alkyl)-carbazol-3-yl, which are substituted by ($C_1$-$C_4$)-alkoxy, halogen, hydroxy and/or di-($C_1$-$C_4$)-alkylamino radicals.

**3.** Compounds as claimed in claim 1 or 2, wherein

A denotes a single bond,

B denotes the -CH = CH- group,

$R_1$ denotes methoxyphenyl, and

$R_2$ denotes p-ethoxyphenyl, p-dimethylaminophenyl, p-diethylaminophenyl, o-chloro-p-dimethylaminophenyl, o-hydroxy-p-diethylaminophenyl or N-ethylcarbazol-3-yl.

**4.** A process for the preparation of a compound of the general formula I according to claim 1, in which,

A denotes a single bond and

B denotes the -CH = CH- group,

the process comprising condensing a compound of the formula II

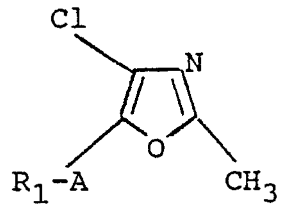

(II)

in which $R_1$ has the indicated meaning, with a compound of the formula III

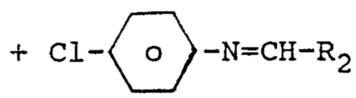

(III)

in which $R_2$ has the indicated meaning, in an inert organic solvent in the presence of a base, at a temperature in the range from 20 °C to 80 °C, separating the compound produced, drying it and optionally purifying it by reprecipitation.

**5.** A process for the preparation of a compound of the general formula I according to claim 1, in which
A denotes the -CH=CH- group and
B denotes a single bond,
the process comprising condensing a compound of the formula IV

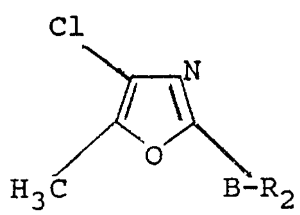

(IV)

in which $R_2$ has the indicated meaning with a compound of the formula V

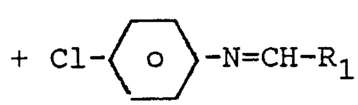

(V)

in which $R_1$ has the indicated meaning, in an inert organic solvent in the presence of a base, at a temperature in the range from 0 °C to 40 °C, separating the compound produced, drying it and optionally purifying it by reprecipitation.

**6.** A process for the preparation of a compound of the general formula I according to claim 1, in which
A denotes a single bond, and
B denotes -CH=CH- or the -CH=CH-CH=CH- group
the process comprising condensing a compound of the formula VI

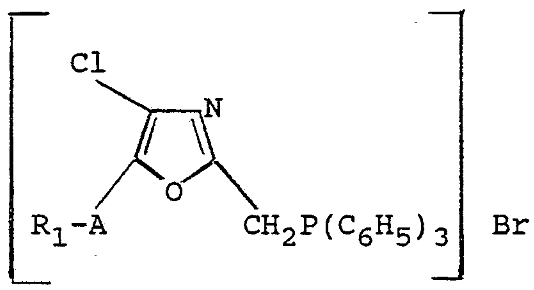

(VI)

in which $R_1$ has the indicated meaning with a compound of the formula VII

X - $R_2$ (VII),

in which $R_2$ has the indicated meaning and X stands for -CHO or -CH=CH-CHO, in an inert organic solvent in the presence of a base, at room temperature, separating the compound produced from the reaction mixture, drying it and optionally purifying it by reprecipitation.

**7.** Use of the compounds of the general formula I as photoconductive substances.

**Revendications**

**1.** Dérivés 4-chloro-oxazole de formule générale I

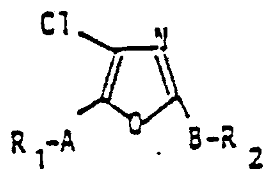

(I)

dans laquelle
- A représente une simple liaison ou le groupe -CH=CH-,
- B représente une simple liaison, le groupe -CH=CH- ou -CH=CH-CH=CH- et est alors une simple liaison lorsque A représente le groupe -CH=CH-,
- $R_1$ représente un groupe phényle qui est éventuellement une ou plusieurs fois substitué par un ou des radicaux alcoxy en $C_1$-$C_4$ ou dialkyl($C_1$-$C_4$)-amino, et
- $R_2$ représente un groupe phényle, phényloxazolyle, pyridyle, julolidine-9-yle, N-alkyl($C_1$-$C_4$)-carbazol-3-yle, coumarine-6-yle ou stilbène-4-yle, qui sont éventuellement une ou plusieurs fois substitués par des radicaux alkyle en $C_1$-$C_4$, alcoxy en $C_1$-$C_4$, halogéno-alcoxy en $C_1$-$C_4$, hydroxy, dialkyl($C_1$-$C_4$)-amino ou dibenzylamino ou par des atomes d'halogène.

**2.** Composés selon la revendication 1,
dans lesquels
- A représente une simple liaison,
- B représente le groupe -CH=CH-,
- $R_1$ représente un groupe phényle qui est substitué par des radicaux alcoxy en $C_1$-$C_4$, et
- $R_2$ représente un groupe phényle ou N-alkyl($C_1$-$C_4$)-carbazol-3-yle qui sont substitués par des atomes d'halogène ou par des radicaux alcoxy en $C_1$-$C_4$, hydroxy et/ou dialkyl-($C_1$-$C_4$)-amino.

**3.** Composés selon la revendication 1 ou 2,
dans lesquels

A représente un simple liaison,
B représente le groupe -CH = CH-,
$R_1$ représente le groupe méthoxyphényle et
$R_2$ représente le groupe p-éthoxyphényle, p-diméthylaminophényle, p-diéthylaminophényle, o-chloro-p-diméthylaminophényle, o-hydroxy-p-diéthylaminophényle ou N-éthylcarbazol-3-yle.

**4.** Procédé pour la préparation d'un composé de formule générale I selon la revendication 1, dans laquelle
A represente une simple liaison et
B représente le groupe -CH = CH-,
dans lequel on condense un composé de formule II

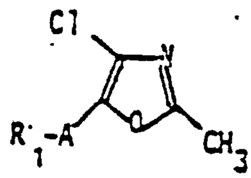

(II)

ayant pour $R_1$ la signification donnée, avec un composé de formule III

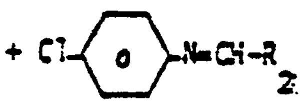

(III)

ayant pour $R_2$ la signification donnée, dans un solvant organique inerte, en présence d'une base, à une température dans la plage de 20 à 80 ° C, on sépare le composé, on le sèche et éventuellement on le purifie par reprécipitation.

**5.** Procédé pour la préparation d'un composé de formule générale I selon la revendication 1, dans laquelle
A représente le groupe -CH = CH- et
B représente une simple liaison,
dans lequel on condense un composé de formule IV

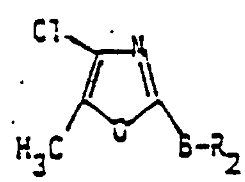

(IV)

ayant pour $R_2$ la signification donnée, avec un composé de formule V

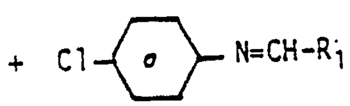

(V)

ayant pour $R_1$ la signification donnée, dans un solvant organique inerte, en présence d'une base, à une température dans la plage de 0 à 40 ° C, on sépare le composé, on le sèche et éventuellement on le purifie par reprécipitation.

**6.** Procédé pour la préparation d'un composé de formule générale I selon la revendication 1, dans laquelle
A représente une simple liaison et
B représente le groupe -CH = CH- ou -CH = CH-CH = CH-,
dans lequel on condense un composé de formule VI

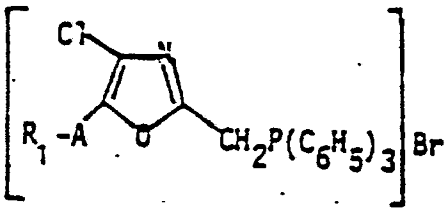

(VI)

ayant pour $R_1$ la signification donnée, avec un composé de formule VII

$X-R_2$ (VII)

ayant pour $R_2$ la signification donnée, et dans laquelle X représente -CHO ou -CH = CH-CHO, dans un solvant organique inerte, en présence d'une base, à la température ambiante, on sépare le composé du mélange réactionnel, on le sèche et éventuellement on le purifie par reprécipitation.

**7.** Utilisation des composés de formule générale I en tant que substances photoconductrices.